# EUROPEAN PATENT SPECIFICATION

(11) **EP 3 745 864 B1**
(45) Date of publication and mention of the grant of the patent: **24.01.2024**
(21) Application number: 19704417.5
(22) Date of filing: 31.01.2019
(51) Int. Cl.: A01N 63/40, A01P 1/00, A23B 4/22, A23B 7/155, C12N 7/00, A23L 3/3571

(54) **BACTERIOPHAGES FOR FOOD DECONTAMINATION**
BAKTERIOPHAGEN ZUR DEKONTAMINATION VON LEBENSMITTELN
BACTÉRIOPHAGES POUR LA DÉCONTAMINATION DES ALIMENTS

(30) Priority: 31.01.2018 GB 201801596
(43) Date of publication of application: 09.12.2020
(73) Proprietor: APS BIOCONTROL LIMITED, Angus DD7 6LE (GB)
(72) Inventor: BLACKWELL, Alison, Angus DD7 6LE (GB); MACK, Kiri, Perth PH14 9TG (GB)
(74) Representative: Marks & Clerk LLP
(86) International application number: PCT/GB2019/050273
(87) International publication number: WO 2019/150121

(56) References cited:
- EP-A1- 1 930 417
- EP-A2- 0 290 295
- HENK R HOOGENKAMP: "Your enemy's foe is your friend", FLEISCHWIRTSCHAFT INTERNATIONAL, no. 2, 1 January 2009 (2009-01-01), pages 44-49, XP055566277, Germany
- Elaine Silva ET AL: "Control of Listeria monocytogenes growth in soft cheeses by bacteriophage P100", Brazilian journal of microbiology : [publication of the Brazilian Society for Microbiology], 1 January 2014 (2014-01-01), pages 11-16, XP055566285, Brazil DOI: 10.1590/S1517-83822014000100003 Retrieved from the Internet: URL:https://www.phageguard.com/wp-content/ uploads/2016/10/Silva-E.-N.-G.-et-al.-2014 .-Control-of-Listeria-monocytogenes-growth -in-soft-cheeses-by-bacteriophage-P100.-Br azilian-Journal-of-Microbi.pdf
- S. GUENTHER ET AL: "Virulent Bacteriophage for Efficient Biocontrol of Listeria monocytogenes in Ready-To-Eat Foods", APPLIED AND ENVIRONMENTAL MICROBIOLOGY, vol. 75, no. 1, 14 November 2008 (2008-11-14), pages 93-100, XP055566292, US ISSN: 0099-2240, DOI: 10.1128/AEM.01711-08
- ZHEN LUO ET AL: "Quantitative real time measurements of bacteria-bacteriophages interactions in fresh lettuce leaves", JOURNAL OF FOOD ENGINEERING, vol. 111, no. 1, 25 January 2012 (2012-01-25), pages 176-185, XP055566282, GB ISSN: 0260-8774, DOI: 10.1016/j.jfoodeng.2011.12.025
- BRITTA LEVERENTZ ET AL: "Examination of Bacteriophage as a Biocontrol Method for Salmonella on Fresh-Cut Fruit: A Model Study", JOURNAL OF FOOD PROTECTION, vol. 64, no. 8, 1 August 2001 (2001-08-01) , pages 1116-1121, XP055566299, US ISSN: 0362-028X, DOI: 10.4315/0362-028X-64.8.1116
- F. MCKENNA ET AL: "Novel in vivo use of a polyvalent Streptomyces phage to disinfest Streptomyces scabies-infected seed potatoes", PLANT PATHOLOGY, vol. 50, no. 6, 1 December 2001 (2001-12-01), pages 666-675, XP055566334, GB ISSN: 0032-0862, DOI: 10.1046/j.1365-3059.2001.00648.x
- ROBERT CZAJKOWSKI: "Bacteriophages of Soft Rot Enterobacteriaceae -a minireview", FEMS MICROBIOLOGY LETTERS, vol. 363, no. 2, 30 November 2015 (2015-11-30), page fnv230, XP055566493, DOI: 10.1093/femsle/fnv230
- GREER G G: "HOMOLOGOUS BACTERIOPHAGE CONTROL OF PSEUDOMONAS GROWTH AND BEEF SPOILAGE", JOURNAL OF FOOD PROTECTION, INTERNATIONAL ASSOCIATION FOR FOOD PROTECTION, US, vol. 49, no. 2, 1 January 1986 (1986-01-01), pages 104-109, XP001024459, ISSN: 0362-028X
- DATABASE WPI Week 201327 Thomson Scientific, London, GB; AN 2013-D35423 XP002789542, & KR 2013 0020710 A (RURAL DEV ADMINISTRATION) 27 February 2013 (2013-02-27)
- .: "Meet & Match Micreos @ 0:42 min of 1:41 min", YOUTUBE VIDEO, 19 February 2015 (2015-02-19), pages 1-1, XP055566610, Internet Retrieved from the Internet: URL:https://www.youtube.com/watch?v=B6G8Fb 70IVI [retrieved on 2019-03-08]
- Alison Blackwell: "The Potential for Bacteriophage to Control Soft Rot Development in Store", AHDB Potato Storage Forum, 1 March 2016 (2016-03-01), pages 1-18, XP055566661, UK Retrieved from the Internet: URL:https://potatoes.ahdb.org.uk/sites/def ault/files/publication_upload/APS%20Biocon trol%20Ltd.pdf [retrieved on 2019-03-08]
- EMAN MAREI ET AL: "Biological Control of Pectobacterium carotovorum Via Specific Lytic Bacteriophage", J. BASIC APPL. SCI. RES, vol. 7, 3 June 2017 (2017-06-03), pages 1-9, XP55566788, ISSN: 2090-4304

## Description

### FIELD OF THE INVENTION

Described herein are methods, compositions and uses which may be applied to the prevention, management and/or control of microorganisms that cause damage, disease, spoilage and/or or loss of food, including vegetable, fruit and other fresh produce. The invention exploits bacteriophages (or "phages") as a means to inactivate, kill or destroy microorganisms present on food and which are associated with, for example, disease, damage and spoilage (rotting).

### BACKGROUND OF THE INVENTION

The presence of certain microorganisms in or on food can be associated with food spoilage, rotting and/or disease. Fruit and vegetables (including root vegetables/tubers and edible (salad) leaves)) are particularly prone to microbial induced rotting and spoilage as in addition to any microorganisms which form part of their natural flora, freshly harvested fruit and vegetables can become contaminated with soil, compost and/or water, all of which may contain undesirable microorganisms, some of those microorgainsms being associated with spoilage and/or rotting of food.

Fruit and vegetables which are stored and/or placed in bags for sale and particularly susceptible to microbial induced spoilage and/or rotting as the atmosphere, for example the humidity during storage and/or in a bag, can promote microbial growth. Furthermore, the harvesting and/or preparation process may cause damage to the food leaving them vulnerable to microbial infection. Additionally, crops and other food plants (or produce) may become contaminated in the field (i.e. while been grown); this contamination may cause the crop/food plant to succumb to diseases. These diseases may spoil the crop/food plant and/or may reduce yield. Seed may also be similarly effected - that is to say, seed may be contaminated with microorganisms (including bacteria); this contamination may occur in storage and/or in the field and can lead to disease in plants grown or germinated therefrom. Food which is prepared for sale and/or storage is subject to washing and other preparation procedures which are aimed at removing dirt, microorganisms and other contaminants. These procedures reduce the risk of the stored or packed food succumbing to microbial induced spoilage and/or rotting. For example, in the case of edible leaves prepared for sale as bagged "ready to use" salads, the leaves are washed prior to bagging. But in the event the water is contaminated, the bagged leaves may quickly spoil and/or rot.

Without wishing to be bound by example, microorganisms of the bacterial genera *Pseudomonas, Acinetobacter, Moraxella, Erwinia, Pectobacterium, Dickeya* and *Escherichia* (and other members of the *Enterobacteriaceae*) and fungal genera *Botrytis, Penicilliumi, Guignardia, Fusarium* and *Sclerotinia* are frequently associated with food disease, spoilage, damage and/or rotting.

Washing and other preparation procedures may greatly reduce instances of spoilage and/or rotting in packaged and/or stored food but there is a need for additional processes which target those microorganisms often present on or in food and which are known to be associated with these problems.

Hoogenkamp describes the use of bacteriophages to treat listeria-contaminated food products ("Your enemy's foe is your friend", Fleischwirtschaft International, no. 2, 1 January 2009, pages 44 to 49). Silva et al describe a study of the effect of bacteriophage P100 on strains of Listeria monocytogenes in artificially inoculated soft cheeses ("Control of Listeria moncytogenes growth in soft cheeses by bacteriophage P100", Brazilian Journal of Microbiology, 1 January 2014, pages 11 to 16). Guenther et al describe a study on the broad-host-range phages A511 and P100 for control of Listeria monocytogenes strains Scott A (serovar 4b) and WSLC 1001 (serovar 1/2a) in different ready-to-eat foods known to frequently carry the pathogen ("Virulent Bacteriophage for Efficient Biocontrol of Listeria monocytogenes in Ready-To-Eat Foods", Applied and Environmental Microbiology, vol. 75, no. 1, 14 November 2008, pages 93 to 100). Luo et al describe a non-invasive real-time imaging approach to quantitatively measure interactions of bacteriophages with both the surface inoculated and the infiltrated model bacterial cells in leafy greens ("Quantitative real time measurements of bacteria-bacteriophage interactions in fresh lettuce leaves", Journal of Food Engineering, vol. 111, no. 1, 25 January 2012, pages 176 to 185). Leverentz et al describe investigations into the effect of lytic, Salmonella-specific phages on reducing Salmonella numbers in experimentally contaminated fresh-cut melons and apples stored at various temperatures ("Examination of Bacteriophage as a Biocontrol Method for Salmonella on Fresh-Cut Fruit: A Model Study", Journal of Food Protection, vol. 64, no. 8, 1 August 2001, pages 1116-1121). Mckenna et al describe an evaluation of a highly virulent and polyvalent Streptomyces phage to disinfest seed potato tubers artificially inoculated with a common scab-causing streptomycte ("Novel in vivo use of a polyvalent Steptomyces phage to disinfest Streptomcyes scabies-infected seed potatoes", Plant Pathology, vol. 50, no. 6, 1 December 2001, pages 666-675). Czajkowski provides a review to summarize recent research on bacteriophages infecting Pectobacterium spp. and Dickeya spp. ("Bacteriophages of Soft Rot Enterobacteriaceae - a minireview", FEMS Microbiology Letters, vol. 363, no. 2, 30 November 2015, page 1 to 6). Greer describes a study on the effect of homologous bacteriophages upon growth of a beef spoilage pseudomonad ("Homologous Bacteriophage Control of Pseudomonas Growth and Beef Spoilage", Journal of Food Protection, International Association for Food Protection, US, Vol. 49, no. 2, 1 January 1986, pages 104-109). KR 2013 0020710 (Rural Dev. Administration) describes a bacteriophage MY-1 (KACC 97009P) capable of preventing bacterial soft rot in vegetables such as Chinese cabbage and lettuce. EP0290295 A (Microbial Dev. Ltd) describes the use of bacteriophages for controlling unwanted fermentation of food-stuffs, especially silage and cheese, by bacteria. EP 1 930417 A (Nymox Pharmaceutical Corp.) describes a bacteriophage composition useful in treating food products to prevent bacterial contamination by *Escherichia coli* bacteria. A YouTube video describes SalmonelexTM and ListexTM P100 that comprise phages and buffered saline. Blackwell describes the potential use of bacteriophage to control potato soft rot development in store ("The Potential for Bacteriophage to Control Soft Rot Development in Store", AHDB Potato Storage Forum, 1 March 2016, pages 1 to 18). Marei et al describe that a lytic bacteriophage was found to control the soft rot disease of potato tubers caused by Pectobacterium carotovorum ("Biological Control of Pectobacterium carotovorum via specific lytic bacteriophage", J. Basic Appl. Sci. Res. vol. 7, 3 June 2017, pages 1 to 9).

### SUMMARY OF THE INVENTION

Aspects of the present invention are set out in the appended claims.

The present disclosure is based on the finding that phages and compositions comprising the same (including composition comprising one or more different phages), may be exploited as a means to decontaminate, purge or cleanse food of microorganisms. The presence of microorganisms in or on food can lead to, for example, disease, damage (loss of food), a shortening of shelf life, premature ripening, reduced yield, spoilage and/or rotting. Microbial contamination of food also represents a potential health risk, increasing the likelihood of food poisoning and the like. This disclosure provides methods, uses and compositions which may be exploited as a means to reduce or eliminate the presence of microorganisms on or in food and in turn, the various problems associated therewith.

As such, a first aspect provides a method of decontaminating food of one or more microorganisms, said method comprising contacting the food with one or more phages, wherein the phages are selected from the group consisting of:
(i) a phage deposited as NCIMB 42290;
(ii) a phage deposited as NCIMB 42292;
(iii) a phage deposited as NCIMB 42294;
(iv) a phage deposited as NCIMB 42296;
(v) a phage deposited as NCIMB 42298;
(vi) a phage deposited as NCIMB 42300;
(vii) a phage deposited as NCIMB 42302;
(viii) a phage deposited as NCIMB 42304;
(ix) a phage deposited as NCIMB 43321;
(x) a phage deposited as NCIMB 43323;
(xi) a phage deposited as NCIMB 43325;
(xii) a phage deposited as NCIMB 43327;
(xiii) a phage deposited as NCIMB 42520;
(xiv) a phage deposited as NCIMB 42522;
(xv) a phage deposited as NCIMB 42524;
(xvi) a phage deposited as NCIMB 42526; and
(xvii) a phage deposited as NCIMB 42528.

A second aspect provides a phage for use in a method of decontaminating food of one or more microorganisms
wherein the phage is selected from the group consisting of:
(i) a phage deposited as NCIMB 42290;
(ii) a phage deposited as NCIMB 42292;
(iii) a phage deposited as NCIMB 42294;
(iv) a phage deposited as NCIMB 42296;
(v) a phage deposited as NCIMB 42298;
(vi) a phage deposited as NCIMB 42300;
(vii) a phage deposited as NCIMB 42302;
(viii) a phage deposited as NCIMB 42304;
(ix) a phage deposited as NCIMB 43321 ;
(x) a phage deposited as NCIMB 43323;
(xi) a phage deposited as NCIMB 43325;
(xii) aphage deposited as NCIMB 43327;
(xiii) a phage deposited as NCIMB 42520;
(xiv) a phage deposited as NCIMB 42522;
(xv) a phage deposited as NCIMB 42524;
(xvi) a phage deposited as NCIMB 42526; and
(xvii) a phage deposited as NCIMB 42528.

A third aspect provides a composition for the decontamination of food of one or more microorganisms, said composition comprising one or more phages, wherein the phages are selected from the group consisting of:
(i) a phage deposited as NCIMB 42290;
(ii) a phage deposited as NCIMB 42292;
(iii) a phage deposited as NCIMB 42294;
(iv) a phage deposited as NCIMB 42296;
(v) a phage deposited as NCIMB 42298;
(vi) a phage deposited as NCIMB 42300;
(vii) a phage deposited as NCIMB 42302;
(viii) a phage deposited as NCIMB 42304;
(ix) a phage deposited as NCIMB 43321;
(x) a phage deposited as NCIMB 43323;
(xi) a phage deposited as NCIMB 43325;
(xii) a phage deposited as NCIMB 43327;
(xiii) a phage deposited as NCIMB 42520;
(xiv) a phage deposited as NCIMB 42522;
(xv) a phage deposited as NCIMB 42524;
(xvi) a phage deposited as NCIMB 42526; and
(xvii) a phage deposited as NCIMB 42528.

A fourth aspect provides food and/or stored or packed food wherein the food has been treated with:
(i) the method of the first aspect;
(ii) the phage of the second aspect; or
(iii) the composition of the third aspect.

The phages or a composition comprising the same, may be useful in decontaminating plants and/or seed.

For the avoidance of doubt, it should be noted that throughout this specification, the terms "comprise" and/or "comprising" are used to denote that aspects and/or embodiments of the invention "comprise" the noted features and as such, may also include other features. However, in the context of this invention, the terms "comprise" and "comprising" encompass embodiments in which the invention "consists essentially of" the relevant features or "consists of" the relevant features.

Food which may be subject to the methods and uses described herein may include any food, food stuff and/or food product for human or animal consumption. The term "food" may embrace any manufactured, harvested, farmed or processed food, food stuff, produce or product. The term food may further be applied to any fresh produce as well as prepared and/or processed produce which has been stored or prepared for storage. For example, the term food (or indeed the term "plants" as used herein) may encompass any plant or animal matter including, for example crops, cereals, dairy produce, meat, vegetables (including root vegetables), edible leaves (eg. herb or salad leaves), fruit, seeds, tubers, bulbs and the like.

The term "food" may encompass, for example, cucumbers, tomatoes, tubers (e.g. potatoes), apples, oranges, bananas, carrots, sprouts, beetroots, turnips, cabbages, edible leaves (salad leaves including all types of lettuce, spinach, rocket, chard, endive, watercress) and any other type of vegetable or fruit. The invention may find application in treating or preventing spoilage and/or rotting of food, including perishable food stuffs. For convenience, the various types of food to which this invention may be applied to shall be collectively referred to as "food".

Food, in particular (freshly) harvested produce from fields is frequently contaminated with microorganisms which, if not removed, may present a risk to health and/or lead to (premature) spoilage of the food through rotting. Indeed, certain microorganisms present in the environment and/or soil, are known to promote rotting or spoilage in vegetables and root vegetables (including edible tubers and leaves) and therefore it would be advantageous to remove these microorganisms, or at least reduce or substantially reduce their presence as contaminants, before packaging these types of food for storage or sale. Thus, prior to, during and/or after processing, packaging and/or storage, fruit and vegetables may be subject to a method or use of this invention or applied a composition of this invention so as to reduce the presence of microbial contaminants which might otherwise promote spoilage and/or rotting if not removed therefrom or destroyed, inactivated or killed.

The term "microorganism" as used herein may include any microorganism which is part of the microbial flora of a particular type of food and/or any food borne microorganism. A food borne microorganism may be any microorganism naturally present in a food or on the surface thereof. Additionally, or alternatively, the term "microorganism" may encompass microbial contaminants or "pathogens" of food. Microbial contaminants of food may be present as surface contaminants and/or as contaminants which have infected and/or penetrated the tissues and/or components of the food. The microbial contamination of food may occur as a consequence of contamination by, for example, soil or (contaminated) water (for example water used to wash during food processing and/or packaging procedures). Food borne microorganisms (contaminants or otherwise) may be associated with spoilage, premature ripening and/or rotting of food - including, for example, perishable food stuffs and/or products, fruit, (root) vegetables and/or edible leaves.

Food, in particular, vegetables and/or edible leaves, may be prepared for sale in pre-packed bags. In addition to any microorganisms naturally present in or on the vegetables and/or edible leaves, this type of food product may contain traces of soil and/or compost which provide a potential source of microbial contamination. While washing and other cleaning procedures may be used to remove microorganisms and potential sources of microbial contamination, microorganisms (contaminants or otherwise) which remain present on or in the food as it is packed, may result in spoilage and/or rotting of the food. Additionally or alternatively, the washing procedures used to prepare and process food (for example vegetables) for bagging, may inadvertently use contaminated water which could introduce a further source of microbial contamination. In addition to inducing spoilage etc., microbial contamination may further represent a potential risk to human health.

Disclosed herein are means (methods, compositions and uses) to facilitate the decontamination of food. The term "decontamination" encompasses any process aimed at reducing or eliminating the numbers of microorganisms (both those which naturally occur on the surface of the food and/or those which are contaminants) present on or in a given type of food. Standard food decontamination processes may comprise one or more washing or cleaning procedures which remove surface dirt and/or perhaps some surface microorganisms and/or microbial contamination. Moreover, fruit and vegetables (including edible leaves) may be further processed to remove bruising and/or other lesions/wounds which might be prone or susceptible to microbial infection/contamination. However, these procedures may not adequately remove microorganisms and thus, the compositions, methods and uses of this invention may be exploited as additional and/or alternative means for achieving the microbial decontamination of food.

It should be understood that the compositions described herein may not only be applied to food as it is processed for packing and/or storage, but also to crops as they are growing and even to seeds before they are planted. Indeed, food seed, crops and/or products, may be regularly treated with compositions of this invention so as to achieve regular decontamination.

In accordance with this invention, the decontamination of microorganisms present on or in food (as contaminants or otherwise) may take the form of the destruction, inactivation and/or killing of one or more microorganisms and/or the inactivation or killing of a proportion (for example about 0.001% to about 1%, about 10%, about 20%, about 30%, about 40%, about 50%, about 60%, about 65%, about 70%, about 75%, about 80%, about 85%, about 90%, about 95%, about 96%, about 97%, about 98%, about 99% or about 100%) of a population of food borne microorganisms.

The microbial flora of any given food (including all those types of food described herein) may comprise isolated cells (individual microorganisms), colonies and/or spores. Additionally or alternatively, the contamination may take the form of one or more populations or biofilms, which populations or biofilms comprise one or more different microorganisms. The term "microbial flora" and any contaminating microorganisms may further comprise one or more microbial species representing, for example, a number of different microbial types. For example, the microbial flora or any microbial contaminant of food may comprise different types, strains and/or species of bacteria, viruses, fungi and/or protozoa. Thus the process of decontamination as described herein may encompass the inactivation, destruction and/or killing of any, all or a proportion (as described above) of the isolated microbial cells, microbial colonies, microbial biofilms and/or microbial populations

Thus, as explained in more detail below, the compositions, methods and uses described herein may exploit one or more phage types specific for one or more of the microorganisms which make up the microbial flora of a food to be decontaminated. The phage types for use in this invention may be specific for one or more microorganisms associated with food damage, loss, spoilage and/or rotting.

The phage used in the methods, compositions and uses described herein may each target one or more types of bacteria.

The phages suitable for use according to this disclosure may be able to target a food borne microorganism (for example a food borne bacteria or food borne bacterial pathogen), in particular, those associated with spoilage, or rotting.

Phages which target certain microorganisms may be said to exhibit an affinity or specificity for the microorganism. Suitable phages may exhibit a specificity and/or affinity for a particular microorganism(s). Without wishing to be bound by theory, phages for use according to this disclosure may infect a microorganism and subsequently (i.e. post infection) destroy, inhibit, kill and/or inactivate that microorganism. Phages suitable for use according to this disclosure may target certain bacteria and/or exhibit a specificity or affinity for those bacteria. In particular the phages may target and/or be specific to or have an affinity for, those bacteria which cause or are associated with, spoilage or rotting in food. Suitable phages may infect and subsequently lyse or otherwise destroy, inactivate or kill their target bacteria.

Microorganisms (bacteria) which are infected by phages may be termed "host" microorganisms - that is they "host" the phages. Thus phages for use in this disclosure may individually (or collectively) exhibit a host specificity which corresponds to those microorganisms known to be associated with food spoilage and/or rotting.

Without wishing to be bound by theory, the phages described herein may be described as either lytic or lysogenic. Lytic phages for use may include those that kill, inactivate and/or inhibit their target bacteria by first injecting their viral DNA or RNA and then replicating to induce microbial or bacterial cell death by, for example, lysis. Useful lysogenic phages may incorporate their nucleic acid (DNA or RNA) into the host and at some later stage (perhaps in response to a stress stimulus, for example UV light exposure) will replicate and cause bacterial cell death.

Phages may be obtained (or are obtainable) by methods which identify phages with an ability to lyse (kill) certain microorganisms - in particular, those microorganisms (bacteria) which are associated with spoilage and/or rotting in food. Microorganisms which are associated with food spoilage and/or rotting may be isolated from samples of soil (or some other culture substrate), plant matter (roots, shoots, fruit, leaves, stems, seeds, buds ), tubers/root vegetables and/or water (water used in the growing of food and/or water used to wash food). Microorganisms associated with rotting and/or spoilage of food may also be isolated from samples of food which exhibit rot/spoilage symptoms. Thus methods which identify phages may comprise, contacting a microorganism with a phage and/or phage composition and determining whether or not the phage and/or phage composition is able to lyse, kill or destroy the microorganism. In such methods, the phage or phage composition may be referred to as a "test phage/composition". The microorganism may be a microorganism isolated from any of the samples described above. Phages which exhibit an ability to lyse (or are lysogenic) or kill/destroy food borne microorganisms and/or food spoilage associated microorganisms, may be used in the methods, uses and compositions described herein.

One of skill will appreciate that variety and geographical origin of a particular food may dictate its microbial flora and/or the type of microorganism most likely to be associated with contamination, infection, spoilage and/or rot. For example, food from one geographical location may be susceptible or prone to disease, rotting and/or spoilage caused or contributed to by one or more microorganism(s), and the same food from another geographical location may be susceptible to disease, rotting and/or spoilage caused by one or more different microorganism(s). By obtaining samples of rotting food, soil, water, plant matter, tubers from around the world, from different varieties (fruit and vegetables ) of the same food and/or from food exhibiting symptoms of different types of spoilage/rot or disease, it should be possible to provide compositions which are not only effective against food spoilage/rotting bacteria from a range of geographic areas, but also useful in treating or preventing spoilage in different varieties of the same food and different types of spoilage, disease and/or rotting (for example spoilage, disease and/or rotting with a variable microbial aetiology). A phage or phage composition may be modified and/or supplemented with additional phages of the same or different families to target the specific microorganisms of each geographical region.

The disclosure may exploit single or multiple phage types, each phage exhibiting a specificity and/or affinity for a single or multiple different microorganism(s). For example, where a particular food exhibits a particular microbial flora or is likely to be affected by (a) specific microbial contaminant(s), then any composition for use may be formulated so as to contain phage types which are specific to that flora and/or those contaminants. In other words, the phage for use may comprise one or more different phage types each with specificity or affinity for one or more of the relevant microorganisms.

The microbial flora of food (in particular fruit, (root) vegetables, edible leaves and the like) may comprise one or more genera of bacteria including (but not limited to) bacteria of the genera *Pseudomonas, Acinetobacter, Moraxella, Erwinia, Pectobacterium, Dickeya* and *Escherichia* (and other members of the *Enterobacteriaceae*)*.*

The disclosure may provide a means to prevent or reduce instances of for example, soft rot or blackleg/bacterial rots as caused by certain bacteria including, for example, those belonging to the genera *Pectobacterium* and *Dickeya.* The disclosure may be applied to the treatment or prevention of soft rot in, for example potatoes by decontamination of potatoes of bacteria of the genera *Pectobacterium* and *Dickeya.*

For example, suitable bacteriophages may inactivate and/or kill one or more target bacteria as described herein, including any which cause or contribute to (or are associated with) rotting and/or instances of soft rot in vegetables, including those belonging to the genera *Pectobacterium* and *Dickeya.*

Phages that are suitable for use may be derived from one or more taxonomic groups. Where the use of bacteriophages is concerned, tailed-phages of the order *Caudovirales* may be used. For example, bacteriophages belonging to the families *Myoviridae, Siphoviridae* and *Podoviridae* may be used. According to the invention, phages within the scope of the second aspect described before (i.e. according to claim 2) are used.

As such, this general disclosure provides a method of decontaminating food of one or more bacteria, said method comprising contacting the food with one or more *Myoviridae, Siphoviridae* and/or *Podoviridae* bacteriophages. According to the invention the method of decontaminating food comprises contacting the food with one or more phages according to the first aspect described before (i.e. the method is according to claim 1). Furthermore, this general disclosure provides phages for use in a method of decontaminating food of one or more bacteria, said method comprising contacting the food with one or more *Myoviridae, Siphoviridae* and *Podoviridae* bacteriophages. According to the invention the phages for use in a method of decontaminating food comprise one or more phages according to the second aspect described before (*i.e.* phages according to claim 2), and said method comprises contacting the food with one or more of these phages according to the first aspect described above (*i.e.* according to claim 1).

The bacterial may include *Pseudomonas, Pectobacterium* and/or *Dickeya* species.

The general disclosure further provides compositions for the decontamination of food of one or more bacteria, said composition comprising one or more bacteriophages selected from the group consisting of *Myoviridae, Siphoviridae* and *Podoviridae* bacteriophages. According to the invention the composition for the decontamination of food comprises one or more phages according to the third aspect described before (i.e. the composition is according to claim 3).

Again, the bacteria against which the composition of this embodiment of the invention may be used, may include *Pseudomonas, Pectobacterium* and/or *Dickeya* species.

This disclosure may provide a method of decontaminating food of one or more bacteria, said method consisting essentially of, or consisting of, contacting the food with one or more *Myoviridae, Siphoviridae* and/or *Podoviridae* bacteriophages. Furthermore, the methods of this disclosure may include methods of decontaminating food of one or more bacteria, which methods consist essentially of, or consist of, contacting the food with one or more *Myoviridae, Siphoviridae* and *Podoviridae* bacteriophages. According to the invention, the pages employed in a method of decontaminating food of one or more bacteria are selected from the group consisting of (i) a phage deposited as NCIMB 42290; (ii) a phage deposited as NCIMB 42292; (iii) a phage deposited as NCIMB 42294; (iv) a phage deposited as NCIMB 42296; (v) a phage deposited as NCIMB 42298; (vi) a phage deposited as NCIMB 42300; (vii) a phage deposited as NCIMB 42302; (viii) a phage deposited as NCIMB 42304; (ix) a phage deposited as NCIMB 43321 ; (x) a phage deposited as NCIMB 43323; (xi) a phage deposited as NCIMB 43325; (xii) a phage deposited as NCIMB 43327; (xiii) a phage deposited as NCIMB 42520; (xiv) a phage deposited as NCIMB 42522; (xv) a phage deposited as NCIMB 42524; (xvi) a phage deposited as NCIMB 42526; and (xvii) a phage deposited as NCIMB 42528.

The disclosure further provides compositions for the decontamination of food of one or more bacteria, said composition consisting essentially of, or consisting of, one or more *Myoviridae, Siphoviridae* and *Podoviridae* bacteriophages. According to the invention, the the compositions for the decontamination of food of one or more bacteria comprise one or more phages selected from the group consisting of (i) a phage deposited as NCIMB 42290; (ii) a phage deposited as NCIMB 42292; (iii) a phage deposited as NCIMB 42294; (iv) a phage deposited as NCIMB 42296; (v) a phage deposited as NCIMB 42298; (vi) a phage deposited as NCIMB 42300; (vii) a phage deposited as NCIMB 42302; (viii) a phage deposited as NCIMB 42304; (ix) a phage deposited as NCIMB 43321 ; (x) a phage deposited as NCIMB 43323; (xi) a phage deposited as NCIMB 43325; (xii) a phage deposited as NCIMB 43327; (xiii) a phage deposited as NCIMB 42520; (xiv) a phage deposited as NCIMB 42522; (xv) a phage deposited as NCIMB 42524; (xvi) a phage deposited as NCIMB 42526; and (xvii) a phage deposited as NCIMB 42528.

The compositions of this invention may be suitable for use preventing or reducing instances of rotting and/or spoilage in fruit and/or vegetables, including, for example, root vegetables, edible tubers (potatoes for example) and edible (salad) leaves. For example, a method of preventing, treating or reducing instances of rot and/or spoilage in food (for example on or in fruits and vegetables) may comprise the use of a composition described herein - said composition comprising one or more of the bacteriophages described herein.

The invention may exploit one or more bacteriophages selected from the group consisting of:
APS Ph 1 (deposited as NCIMB 42290)
APS Ph 5 (deposited as NCIMB 42298)
APS Ph 7 (deposited as NCIMB 42302)

For example, a composition of this invention may comprise one, two or all three of the bacteriophages listed as (i), (ii) and (iii) above.

A composition according to the invention exploits one or more of the bacteriophages listed in tables 1 and 3a below. For example, a composition according to the invention may comprise one or more of the bacteriophages listed as (1)-(xiii) below.
(i) APSPb1
(ii) APSPb2
(iii) APSPb3
(iv) APSPb4
(v) APSPb5
(vi) APSPb6
(vii) APSPb7
(viii) APSDs1
(ix) APSPs1
(x) APSPs2
(xi) APSPs3
(xii) APSPs4
(xiii) APSPs5

Additionally or alternatively, a composition according to the invention may exploit one or more of the bacteriophages listed in table 3b below. For example, a composition according to the invention may comprise one or more of the bacteriophages listed as (1)-(4) below.
(1) APSPH9;
(2) APSPH10;
(3) APSPH11; and
(4) APSPH12.

Without wishing to be bound by theory, the inventors have discovered that compositions comprising one or more of the phages according to claim 2 are particularly useful as the microorganisms (for example bacteria) to which they are specific do not mutate sufficiently over time to render the composition useless and/or there are likely multiple attachment sites for the bacteriophages on the bacterial surface.

A composition of this disclosure may further comprise an excipient, diluent or buffer. For example, a composition may comprise an amount of a formulated "phage buffer". The phages for use (one or more of those described herein) may be suspended in a volume of a suitable phage buffer. A useful phage buffer may comprise a buffer and one or more salts (for example inorganic salts). For example, a useful phage buffer may comprise a volume of Tris, an amount of NaCl and MgSO₄.

A suitable formulation may be:
50mM Tris (pH 7.5);
100mM NaCl; and
8mM MgSO₄.

The method, use and/or composition according to the invention may exploit at least two or at least three bacteriophages selected from the group consisting of bacteriophages (i) to (xiii) listed above and in Tables 3a and 3b below. For example, the various uses, methods and compositions of the invention may exploit a "core phage mixture" comprising (or "consisting essentially" or "consisting" of), for example, bacteriophages APSPb1, APSPb2, APSDs1, APSPs1.

In addition to any core or bacteriophage mixtures described herein, the invention may further exploit one or more other phages/bacteriophages, including, for example one or more of the bacteriophages listed as (iii)-(vi), (viii) and (x)-(xiv). Phages or bacteriophages which are combined for use with a core phage/bacteriophage mixture of this invention may be referred to as "supplementary" phages/bacteriophages.

By way of example, a composition of this invention may comprise four (for example the four bacteriophages identified as the "core-mix") bacteriophages.

Alternatively, a composition of this invention may comprise, consist essentially of consist of, an amount of each of the following bacteriophages:
(i) APSPb1 (deposited as NCIMB 42290, 24^{th} September 2014*);
(ii) APSPb5 (deposited as NCIMB 42292, 24^{th} September 2014*);
(iii) APSPb2 (deposited as NCIMB 42294, 24^{th} September 2014*);
(iv) APSPb6 (deposited as NCIMB 42296, 24^{th} September 2014*);
(v) APSPb3 (deposited as NCIMB 42298, 24^{th} September 2014*);
(vi) APSPb4 (deposited as NCIMB 42300, 24^{th} September 2014*);
(vii) APSPb7 (deposited as NCIMB 42302, 24^{th} September 2014*); and
(viii) APSDs1 (deposited as NCIMB 42304, 24^{th} September 2014*).

*All deposits made with the National Collection of Industrial Food and Marine Bacteria (NCIMB) Ltd., of Ferguson Building, Craibstone Estate, Bucksburn, Aberdeen, AB21 9YA.

All deposits (NCIMB 42290, NCIM 42292, NCIMB 42294 and NCIMB 42296, NCIMB 42298, NCIMB 42300, NCIMB 42302, NCIMB 42304) made 14th September 2014.

A composition comprising all eight of the abovementioned phages may be referred to as an "UK mix" or "eight phage mix".

Further details regarding each of these phage types are provided in the table below.

**Table 1**

| **Bacteriophage Host** | | | | **Isolate Details** | | **Bacteriophage** | | **Accession Numbers** | | |
|---|---|---|---|---|---|---|---|---|---|---|
| Lab Designation | Species | Patent Designation | | Location | Sample Type | Lab Designation | Patent Designation | **Phage** | **Host** | **Original code** |
| | *Pectobacterium* | APS Ph1 | | | Potato | | | NCIMB | NCIMB | |
| 1039 | *atrosepticum Pectobacterium* | Host APS Ph2 | | Scotland N. | stem | BRA1 10391 | APS Ph1 | 42290 NCIMB | 42291 NCIMB | APSPb1 |
| 29 | *atrosepticum Pectobacterium* | Host APS Ph3 | | Ireland | Soil | BRA3 29 1 | APS Ph2 | 42292 NCIMB | 42293 NCIMB | APSPb5 |
| BA120521s | *atrosepticum* | Host | 12/05/ | Scotland | water | BA120521s-1 | APS Ph3 | 42294 | 42295 | APSPb2 |
| BS170412- | *Pectobacterium* | APS Ph4 | | | | | | NCIMB | NCIMB | |
| 11 | *atrosepticum Pectobacterium* | Host APS Ph5 | 17/04/2012 | Scotland | water Potato | BL210615-1 | APS Ph4 | 42296 NCIMB | 42297 NCIMB | APSPb6 |
| 103 | *carotovorum Pectobacterium* | Host APS Ph6 | | Scotland | Tuber | wb3 tp103 1 | APS Ph5 | 42298 NCIMB | 42299 NCIMB | APSPb3 |
| AB220313 | *atrosepticum Pectobacterium* | Host APS Ph7 | 22/03/2013 | Scotland | water | AB13 1 | APS Ph6 | 42300 NCIMB | 42301 NCIMB | APSPb4 |
| BL1108-93 | *sp.* | Host APS Ph8 | 11/08/2010 | UK | water Potato | 93.9 | APS Ph7 | 42302 NCIMB | 42303 NCIMB | APSPb7 |
| D. solani 4 | *Dickeya solani* | Host | 2009 | Non-UK | stem | DS4 | APS Ph8 | 42304 | 42305 | APSDs1 |

Another composition of this invention may comprise, consist essentially of consist of, an amount of each of the following bacteriophages:
(i) APS PH9 (Accession no: NCIMB 43321*);
(ii) APS PH10 (Accession no: NCIMB 43323*);
(iii) APS PH11 (Accession no: NCIMB 43325*); and
(iv) APS PH12 (Accession no: NCIMB 43327*).

*All deposits made with the National Collection of Industrial Food and Marine Bacteria (NCIMB) Ltd., of Ferguson Building, Craibstone Estate, Bucksburn, Aberdeen, AB21 9YA. All deposits (NCIMB 43321, NCIM 43323, NCIMB 43325 and NCIMB 43327) made 19^{th} December 2018.

A composition of this type may be referred to as a "European phage mix". The European phage mix may optionally be supplemented with any other phage type described herein. For example, a composition may comprise the "European mix" described above with amounts on one or more phages selected from the group consisting of:
(i) APS Ph3; and
(ii) APS Ph7.

Without wishing to be bound by theory, certain combinations of phages (bacteriophages) may be particularly useful and/or exhibit a synergistic effect. That is to say, that the decontaminating effect of a combination of different phage types is greater than the sum of the decontaminating effect of the individual phage types. For example, the eight phages listed above can be used in an exemplary composition.

The disclosure may further comprise compositions comprising one or more bacteriophages selected from the group consisting of the bacteriophages listed as (i)-(xiii) above. The invention may further provide compositions comprising at least two or at least three of the bacteriophages listed as (i)-(xiii) above.

Compositions of this invention may comprise a core mixture comprising (or "consisting essentially" or "consisting" of) bacteriophages APSPb1, APSPb2, APSDs1, APSPs1.

The compositions of this invention may be formulated for application to food, to plants (food plants) and or to seed. The composition may be provided as liquid formulations which may be poured, sprayed, dropped or wiped onto a food, plant and/or seed to be decontaminated.

The number of bacteriophages present in a composition of this invention may be dictated by the likely or predicted number, diversity and/or type of microorganisms (or microbial flora) present on the food to be decontaminated. The more diverse the microbial flora or contaminants (comprising for example different species or types of bacteria) the more phages may be required to ensure a suitable level of decontamination is achieved. Nevertheless, this invention may be applied equally to the decontamination of a food item of one particular type of microorganism as it can to the decontamination of a food item of two or more different types or strains of microorganism. Thus the term "composition" as used herein, encompasses compositions comprising one phage type or two or more phage types.

A composition of this invention may comprise any suitable concentration or amount of phage. For example the total phage content may be 10² pfu/ml, 10³ pfu/ml, 10⁴ pfu/ml, 10⁵ pfu/ml, 10⁶ pfu/ml, 10⁷ pfu/ml, 10⁸ pfu/ml or 10⁹ pfu/ml 10¹⁰. For example, suitable concentrations may be between about 10⁴ pfu/ml and about 10⁸ pfu/ml.

The compositions of this invention may be used during food processing procedures as a supplement to standard washing, cleaning and/or decontamination techniques. For example, a food may be washed or cleaned in a composition of this invention, which composition comprises one or more phages (for example bacteriophages) of the type described herein. Additionally, or alternatively, prior to packaging or bagging (for example type of fruit, vegetable or edible leaves), a composition of this invention (which composition comprises one or more bacteriophages) may be applied (by dropping, spraying or the like) to the food or food plant/seed.

The method may further comprise a method of preparing food for storage or packing, said method comprising contacting a food to be stored or packed with one or more phage capable of inactivating, killing or destroying one or more microorganisms of the food microbial flora and/or a composition of this invention.

It should be understood that the term "contact" may encompass contact between one or more phages and/or a composition of this invention under conditions which facilitate the infection of the relevant microorganisms with or by the phages. The conditions may also favour the replication of the phages within its host and/or the subsequent inactivation, lysis or killing thereof.

### DETAILED DESCRIPTION

The present invention will now be described in detail with reference to the following figures which show:
Figure 1: Factory success data: *Pectobacterium* spp. contamination of tubers is reduced following bacteriophage treatment.
Figure 2: Application of a bacteriophage mix to tubers prior to planting prevents blackleg formation during growth.
Figure 3: reducing seed-derived blackleg with bacteriophage. *: significant reduction compared with positive control (F_{4,25} = 39.12 *P <* 0.001,n = 6).
Figure 4: *Pectorbacterium* loading in stolon end tissue of individual and combined FG₂ Rudolph samples by Q-PCR (copy number/g). Rudolf PB2 seed treated with the eight phage mix of this disclosure at planting. Bacteriophage applied with a hand mister & allowed to dry before planting. Randomised block- 4 blocks, 1 plot of each treatment per block (98 tubers/plot). 3 trials completed. Seed contamination levels measured pre-planting
Figure 5: % Blackleg plants/plot (n=4). Bacteriophage treatment cannot make completely reverse the effects of contaminated seed but can reduce the expression of disease if environmental conditions are suitable. *: significant reduction compared with untreated control (F_{3,12} = 4,68 *P* = 0.02,n = 4)
Figure 6: PB2 seed (Rudolf) (0% Pba). 4 bacteriophage treatments (control, in furrow (3L product/ha, in 80L, Foliar (3L product/ha, in 200L, applied for 9 weeks from rosette stage) and in furrow & foliar). Randomised block- 4 blocks, 1 plot of each treatment per block with 98 tubers per plot. Infector plants to increase the likelihood of disease symptoms. qPCR analysis on 3 tubers/each of 10 plants/plot. *: significant reduction compared with untreated control (F_{2,42} = 6.35, *P <* 0.001 ,n = 40).
Figure 7: Replanted material from 2017 trial. Data represents observations only - no treatments. 50 tubers were planted per treatment. qPCR analysis on 20 groups of 15 tubers per treatment. *: significant reduction compared with untreated control (Mann Whitney tests, *P* < 0.05).
Figure 8: Species distribution of the 248 *Pectobacterium* species isolated from potato material/potato wash water from The Netherlands and assessed for phage susceptibility in Figure 9: ***Pcc**: Pectobacterium carotovorum* subsp. *carotovorum, **Pcbr** :Pectobacterium carotovorum* subsp. *brasiliense* and ***Ppar.** Pectobacterium parmentieri.Lytic* activity of the eight phage mix and a new 6-phage mix against the isolated Dutch *Pectobacterium* spp. Figure 10: current UK Bacteriophage mix (the "8 phage mix") vs new European mix: % coverage of Pectobacterium spp.

### Selective Bacteriophage Combinations for managing Spoilage Microorganisms of Fresh Produce

Bacteriophage compositions may contain the core bacteriophage composition APSPb1, APSPb2, APSDs1, APSPs1. Some bacteriophage compositions can contain additional bacteriophages to increase the efficacy of the core bacteriophage composition in certain samples, for example in samples from a range of geographic areas, farms and processing factories as well as samples of different types and/or varieties of food.

Phage (for example bacteriophage) mixtures for use in this invention are selected by their ability to lyse (kill) bacteria isolated from samples (soil, plants, tubers, water) which exhibit rot symptoms.

Bacteriophage compositions may be used to target potato soft rots caused by *Pectobacterium* & *Dickeya* spp., and/or in potato processing factories (post-harvest). The bacteriophage compositions could also be used in the field or in potato stores to prevent blackleg/bacterial rots.

Bacteriophage compositions may be used to decontaminate salad produce (including washed and bagged salad leaves) of microorganisms which induce rot, including, for example, rots (such as blackleg) caused by *Pseudomonas* spp. and *Pectobacterium spp.*

Indeed, the various phage compositions described herein may be used to decontaminate a wide range of fresh produce of microorganisms which are associated with rot and spoilage. The compositions of this invention which comprise bacteriophage mixtures may have increased activity, when compared to individual bacteriophage. Some bacteriophage may act synergistically.

The compositions of this invention may be provided as liquid formulations comprising buffer(s) and/or salts. The compositions may be diluted for use.

**Table 2- Composition of exemplar bacteriophage mixtures**

| Target bacterial genus | Core bacterioph age | Supplement ary bacteriopha ge 1 | Supplement ary bacteriopha ge 2 | Supplement ary bacteriopha ge 3 | Supplement ary bacteriopha ge 4 | Supplement ary bacteriopha ge 5 |
|---|---|---|---|---|---|---|
| Pectobacteri um | APSPb1 | APSPb3 | APSPb4 | APSPb5 | APSPb6 | APSPb7 |
| Pectobacteri um | APSPb2 | | | | | |
| Dickeya | APSDs1 | APSDs2 | | | | |
| Pseudomon as | APSPs1 | APSPs2 | APSPs3 | APSPs4 | APSPs5 | |

**Table 3a - Species targeted by each bacteriophage and microbiological deposit information for bacteriophage (and their hosts) employed in the examples and in compositions of this invention.**

| | | Accession Numbers | | Isolate Details | |
|---|---|---|---|---|---|
| **Bacterial Species Targeted** | **Phage code** | **Phage** | **Host** | **Location** | **Sample type** |
| *Pectobacterium atrosepticum* | APSPb1 (APS Ph 1) | NCIMB 42290* | NCIMB 42291* | Scotland | Potato stem |
| *Pectobacterium atrosepticum* | APSPb5 (APS Ph 2) | NCIMB 42292* | NCIMB 42293* | N. Ireland | Soil |
| *Pectobacterium atrosepticum* | APSPb2 (APS Ph 3) | NCIMB 42294* | NCIMB 42295* | Scotland | water |
| *Pectobacterium atrosepticum* | APSPb6 (APS Ph 4) | NCIMB 42296* | NCIMB 42297* | Scotland | water |
| *Pectobacterium atrosepticum* | APSPb3 (APS Ph 5) | NCIMB 42298* | NCIMB 42299* | Scotland | Potato Tuber |
| *Pectobacterium atrosepticum* | APSPb4 (APS Ph 6) | NCIMB 42300* | NCIMB 42301* | Scotland | water |
| *Pectobacterium sp.* | APSPb7 (APS Ph 7) | NCIMB 42302* | NCIMB 42303* | UK | water |
| *Dickeya solani* | APSDs1 (APS Ph 8) | NCIMB 42304* | NCIMB 42305* | Non-UK | Potato stem |
| *Pseudomonas fluorescens* | APS Ps1 (APS PFΦ1) | NCIMB 42520^{y} | NCIMB 42519^{y} | UK | Bagged salad leaves |
| *Pseudomonas fluorescens* | APS Ps2 (APS PFΦ2) | NCIMB 42522^{y} | NCIMB 42521^{y} | UK | Bagged salad leaves |
| *Pseudomonas fluorescens* | APS Ps3 (APS PFΦ3) | NCIMB 42524^{y} | NCIMB 42523^{y} | UK | Bagged salad leaves |
| *Pseudomonas fluorescens* | APS Ps4 (APS PFΦ4) | NCIMB 42526^{y} | NCIMB 42525^{y} | UK | Bagged salad leaves |
| *Pseudomonas fluorescens* | APS Ps5 (APS PFΦ5) | NCIMB 42528^{y} | NCIMB 42527^{y} | UK | Bagged salad leaves |

| | | | | | |
|---|---|---|---|---|---|
| *All deposits made with the National Collection of Industrial Food and Marine Bacteria (NCIMB) Ltd., of Ferguson Building, Craibstone Estate, Bucksburn, Aberdeen, AB21 9YA. All deposits (NCIMB 42290, NCIMB 42291, NCIM 42292, NCIMB 42293, NCIMB 42294, NCIMB 42295, NCIMB 42296, NCIMB 42297, NCIMB 42298, NCIMB 42299, NCIMB 42300, NCIMB 42301, NCIMB 42302, NCIMB 42303, NCIMB 42304 and NCIMB 42305) made 24^{th} September 2014. ^{y}All deposits made with the National Collection of Industrial Food and Marine Bacteria (NCIMB) Ltd., of Ferguson Building, Craibstone Estate, Bucksburn, Aberdeen, AB21 9YA. All deposits (NCIMB 42519, NCIMB 42520, NCIMB 42521, NCIM 42522, NCIMB 42523, NCIMB 42524, NCIMB 42525, NCIMB 42526, NCIMB 42527 and NCIMB 42528) made 27^{th} January 2016. | | | | | |

**Table 3b**

| | | **Accession numbers** | |
|---|---|---|---|
| **Bacterial Species Targeted** | **Phage code** | **Phage** | **Host** |
| *Pectobacterium sp.* | APS PH9 | NCIMB 43321* | NCIMB 43322* |
| *Pectobacterium brasiliense* | APS PH10 | NCIMB 43323* | NCIMB 43324* |
| *Pectobacterium parmentieri* | APS PH11 | NCIMB 43325* | NCIMB 43326* |
| *Pectobacterium carotovorum* | APS PH12 | NCIMB 43327* | NCIMB 43328* |

| | | | |
|---|---|---|---|
| *All deposits made with the National Collection of Industrial Food and Marine Bacteria (NCIMB) Ltd., of Ferguson Building, Craibstone Estate, Bucksburn, Aberdeen, AB21 9YA. All deposits (NCIMB 43321, NCIM 43323, NCIMB 43325 and NCIMB 43327) made 19^{th} December 2018. | | | |

### Methods

### Bacterial isolation:

Samples (typically wash water from processing factories or rotten tubers/plant material) are processed by producing a homogenous suspension of bacteria in buffer, serially diluting and plating on selective microbiological media (crystal violet pectate medium for *Pectobacterium spp.* and King's B medium for *Pseudomonas* spp.). Liquid samples are diluted directly and solid samples are pulsified in buffer (MRD) before dilution. Bacteria are identified using standard microbiological tests (growth or appearance on selective agar, Gram staining etc.) and confirmed by PCR. Selected bacterial colonies are purified and used for subsequent bacteriophage screening.

### Bacteriophage isolation:

A range of environmental samples (inc. soil, water, plant material, digestate from waste processing, sewage, animal stomach contents etc.) were screened for the presence of bacteriophage by enrichment of the environmental samples with the target bacteria in liquid microbiological media. Following enrichment, samples are centrifuged or filtered to remove solid components and bacteria before being spotted against the target bacterial strains. A classic over-lay technique, where the target bacteria are added to a layer of top agar overlaying an agar plate, provides a lawn on which bacteriophage enrichment mixes are spotted and instances of lysis (clearing of the bacteria) can be observed following overnight incubation at room temperature. Enrichment mixes which lyse the target bacteria are selected and individual bacteriophage can be isolated, picking individual plaques in top agar.

### Examples

### Example of tubers of a single variety, from multiple growers

**Table 4-Testing of Bacteriophage mixes against Pectobacterium spp. isolated from tubers supplied from multiple growers**

| Locati on of growe rs | Varie ty | % bacteria lysed by core phage | % bacteria lysed by core phage + APSPb3 | % bacteria lysed by core phage + APSPb4 | % bacteria lysed by core phage + APSPb5 | % bacteria lysed by core phage + APSPb6 | % bacteria lysed by core phage + APSPb7 |
|---|---|---|---|---|---|---|---|
| Scotia nd | Purpl e maje sty | 85% | 85% | 100% | 85% | 85% | 85% |
| Scotia nd | Desir ee | 77% | 77% | nd | 77% | 85% | 85% |
| E. Engla nd | Maris Piper | 60% | 60% | nd | 60% | 90% | 80% |
| Scotia nd and SW. Engla nd | King Edw ard | 0% | 29% | nd | 0% | 43% | 100% |

### Example of different varieties in the same factory

**Table 5: Testing of Bacteriophage mixes against Pectobacterium spp. isolated from tubers supplied from a single processing factory and tested against the core or supplemented core bacteriophage mixes**

| Locati on of grower s | Maris Piper | % bacteria lysed by core bacterioph age compositio n | % bacteria lysed by core bacteriopha ge compositio n + n supplement ary APSPb3 | % bacteria lysed by core bacteriopha ge compositio n + supplement ary APSPb4 | % bacteria lysed by core bacteriopha ge compositio n + supplement ary APSPb5 | % bacteria lysed by core bacteriopha ge compositio + n + supplement ary APSPb6 | % bacteria lysed by core bacteriopha ge compositio n + supplement ary APSPb7 |
|---|---|---|---|---|---|---|---|
| Scotia nd | Purpl e majes ty | 60% | 60% | nd | 60% | 60% | 60% |
| Scotia nd | Desir ee | 77% | 77% | nd | 77% | 85% | 85% |

### Example of same variety in different locations

**Table 6: Testing of Bacteriophage mixes against Pectobacterium spp. isolated from a single variety of tubers supplied from two potato processing factories**

| Locati on of grower s | Varie ty | % bacteria lysed by core bacterioph age compositio n | % bacteria lysed by core bacteriopha ge composition + supplement ary APSPb3 | % bacteria lysed by core bacteriopha ge composition + supplement ary APSPb4 | % bacteria lysed by core bacteriopha ge composition + supplement ary APSPb5 | % bacteria lysed by core bacteriopha ge composition + supplement ary APSPb6 | % bacteria lysed by core bacteriopha ge composition + supplement ary APSPb7 |
|---|---|---|---|---|---|---|---|
| Scotia nd | Maris Piper | 70% | 70% | nd | 70% | 70% | 70% |
| E. Englan d | Maris Piper | 60% | 60% | nd | 60% | 90% | 80% |

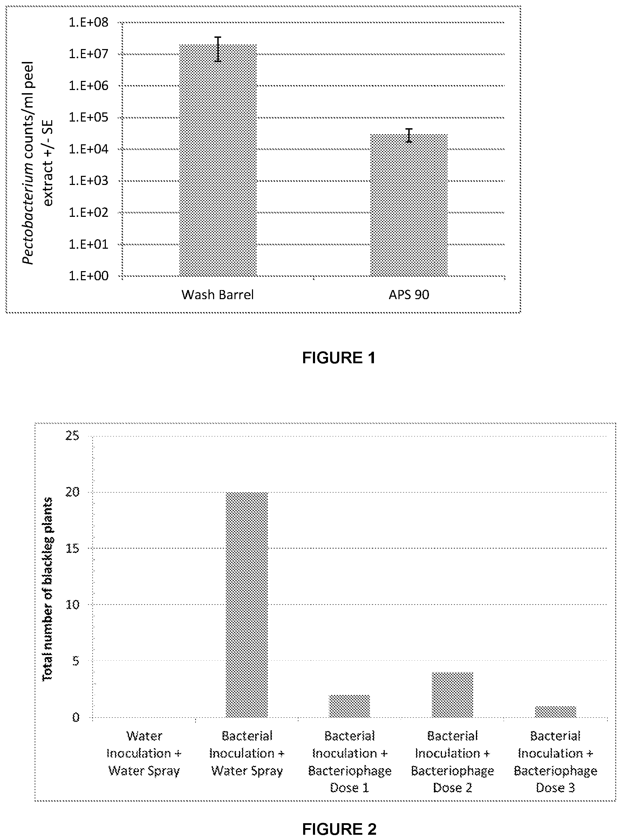

### Factory success data - Figure 1

Tubers were washed in water and either left with no treatment (wash barrel) or sprayed with a bacteriophage composition APS90 containing core bacteriophage + APSPb3 & APSPb5 at 10⁶ pfu/ml. Numbers of *Pectobacterium* spp. were enumerated from tubers after 5 days storage at room temperature.

Data presented in Figure 1 shows a 654-fold reduction in bacterial numbers in tubers treated with the bacteriophage composition APS90.

### Storage trial data

### (a) Prevention of rots during storage

Maris Piper tubers were treated with either no treatment, a water or bacteriophage spray (core + APSPb5 at 10⁶pfu/ml). The tubers were assessed for rot development after 1, 3 and 6 months cold storage at ~2.8°C.

At each assessment tubers were visually inspected for the presence of rots and when found, rotten tubers were removed and planted in glasshouses to determine blackleg levels in daughter plants (see part b). Numbers represent cumulative number of rots identified across all assessment points.

**Table 7: Prevention of rots in tubers treated with bacteriophage prior to cold storage.**

| Treatment | | Month 1 rots | Month 3 rots | Month 6 rots |
|---|---|---|---|---|
| 1 | No Treatment | 9 | 13 | 13 |
| 2 | Water spray | 10 | 11 | 17 |
| 3 | Bacteriophage spray | 9 | 10 | 11 |

The number of rotten tubers treated with bacteriophage spray was lower than that of tubers stored without treatment or treated with a water spray. The difference in the number of rots is greater at longer storage times. This indicates that adding bacteriophage to tubers can increase the shelf life of said tubers when they are stored over a period of time.

### (b) Prevention of blackleg once planted

Maris Piper tubers were treated with either no treatment, water or bacteriophage (core + APSPb5) spray at 10⁶pfu/ml and stored for 1, 3 or 6 months at ~2.8°C. Tubers from part a) of the assessment were planted in glasshouses to determine blackleg levels in daughter plants. No effect of the bacteriophage was observed on emergence of the plants but stem number increased with phage treatment.

**Table 8: Prevention of blackleg by application of bacteriophage mixes to mother tuber prior to cold storage (1, 3 or 6 months pre-planting)**

| | **Emergence** | | | **Total number of stems** | | |
|---|---|---|---|---|---|---|
| Duration between treatment and planting | **No treatment** | **Water Spray** | **Bacteriopha ge spray** | **No treatment** | **Water Spray** | **Bacteriopha ge spray** |
| After 1 month of storage | 23 | 24 | 24 | 32 | 24 | 27 |
| After 3 months of storage | 24 | 24 | 24 | 55 | 50 | 65 |
| After 6 months of storage | 24 | 21 | 24 | 110 | 105 | 115 |

### Treatment of seed immediately before planting

### Pectobacterium spp. example on tubers

Variety: Desiree were damaged and inoculated with *water (negative control) or Pectobacterium spp.* (10⁶cfu/ml, all other treatments) to contaminate the seed potatoes. Immediately prior to planting bacteriophage mixes (core + APSPb3, APSPb4 & APSPb5 at 10⁶ (Dose 1), 10⁷ (Dose 2) or 10⁸ (Dose 3) pfu/ml) were sprayed onto tubers and planted. The number of plants which developed blackleg was assessed following emergence.

### Dickeya spp. example on tubers

Tubers (Variety: Desiree) were inoculated with water (negative control) or Dickeya spp. (107 cfu/ml, all other treatments) to contaminate the seed potatoes. Immediately prior to planting, bacteriophage (core APSDs1 at 107 cfu/ml) was sprayed onto tubers and the treated tubers were planted. The number of plants which successfully emerged was assessed as a measure of disease prevention.

### Selection of a bacteriophage composition suitable for salad processing

### Pseudomonas example - salad leaves

*Pseudomonas* spp. bacteria were isolated from bagged salad samples from a UK salad processor. Bacteriophage were then isolated and tested against these isolates and the proportion of lysis calculated.

**Table 9 - Selecting a bacteriophage mix suitable for salad processing.**

| Sampl e | % bacteria lysed by core bacteriopha ge composition | % bacteria lysed by core bacteriophag e composition + supplementa ry APSPs2 | % bacteria lysed by core bacteriophag e composition + supplementa ry APSPs3 | % bacteria lysed by core bacteriophag e composition + supplementa ry APSPs4 | % bacteria lysed by core bacteriophag e composition + supplementa ry APSPs5 | % bacteria lysed by core bacteriophag e composition + supplementa ry APSPs2 and APSPs3 |
|---|---|---|---|---|---|---|
| Salad mix 1 | 30% | 36% | 36% | 35% | 33% | 41% |
| Salad mix 2 | 16% | 20% | 18% | 17% | 17% | 20% |

A proportion of the Pseudomonas bacteria present in bagged salad leaves was lysed by the core bacteriophage APSPs1. However, adding one additional supplementary bacteriophage (APSPs2, APSPs3, APSPs4 or APSPs5) to the core bacteriophage composition leads to an increase in the efficacy of the treatment, measured in terms of increased % of lysed bacteria. Adding two supplementary bacteriophage to the core simultaneously leads to a greater increase in efficacy (at least for Salad Mix 1) compared to the treatment with core alone or core + a single supplementary phage. This indicates that there may be a synergistic effect in the efficacy of bacteriophage mixtures in lysing bacteria when multiple bacteriophage are used concurrently.

### Reduction in blackleg symptoms

### Reducing seed-derived blackleg with bacteriophage. 6

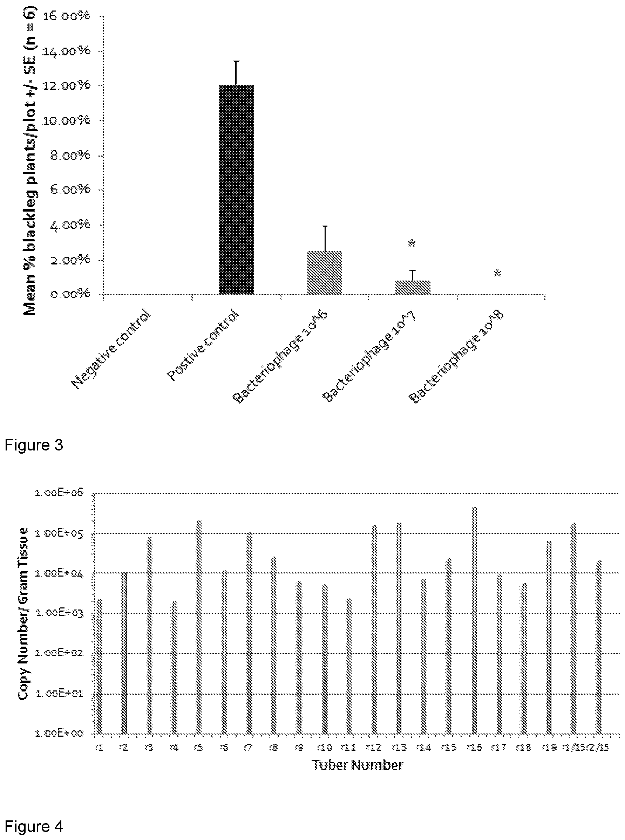

Figure 3 shows Desiree seed treated damaged & inoculated with Pba (10 cfu/ml) and sprayed (until visibly damp) with bacteriophage the eight phage mix of this disclosure at final concentrations of 10⁶ , 10⁷ & 10⁸ pfu/ml. Results show a significant reduction in seed-derived blackleg compared with positive control. Thus Blackleg may be reduced if seed is externally contaminated.

Figure 4 shows *pectobacterium* loading in stolon end tissue of individual and combined FG₂ Rudolph samples by Q-PCR (copy number/g). Rudolf PB2 seed was treated with the eight phage mix at planting. Bacteriophage applied with a hand mister & allowed to dry before planting. Randomised block- 4 blocks, 1 plot of each treatment per block (98 tubers/plot). Seed contamination levels measured pre-planting.

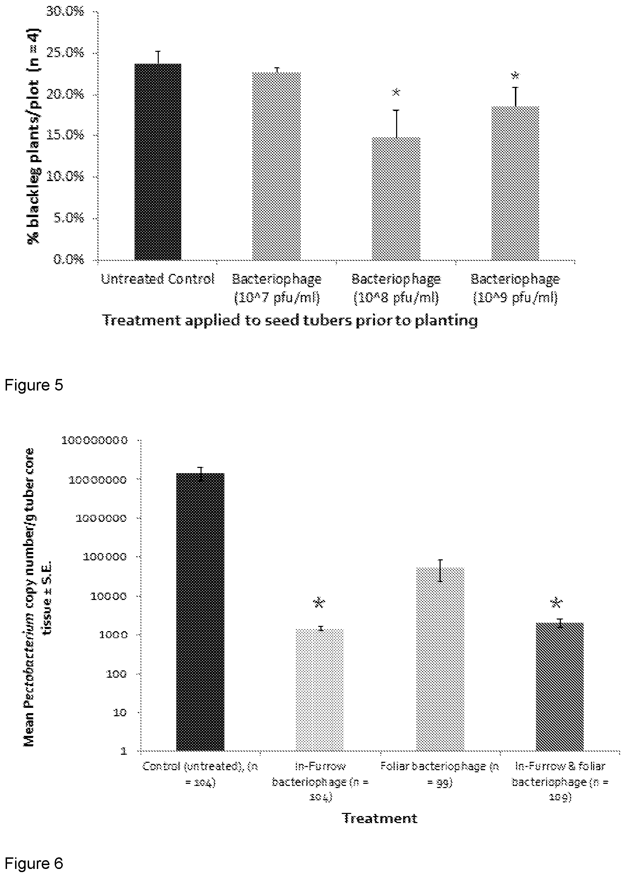

Figure 5 shows that there was a significant reduction in the % blackleg plants/plot and that while Bacteriophage treatment cannot completely reverse the effects of contaminated seed, it can reduce the expression of disease if environmental conditions are suitable.

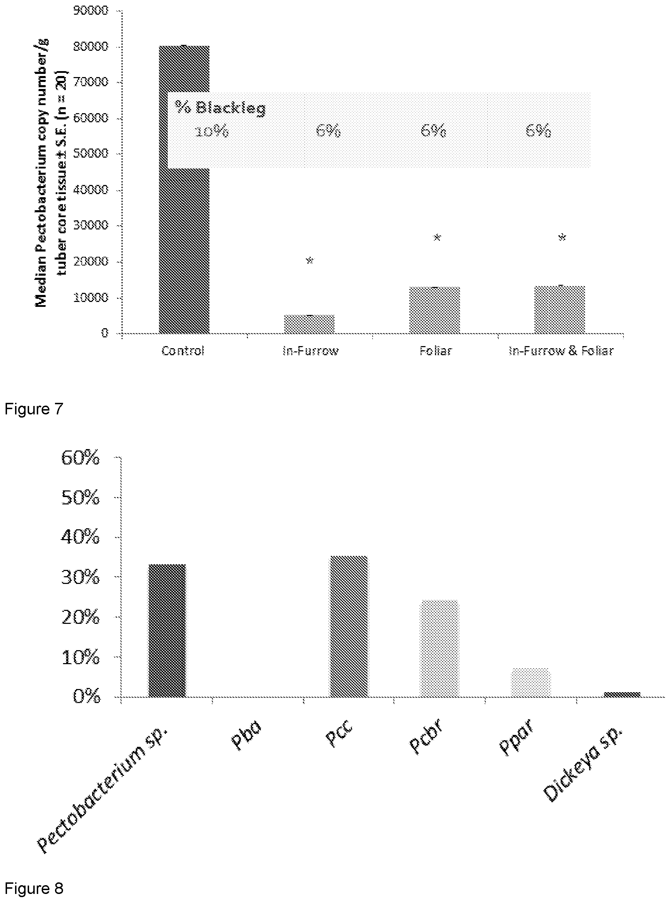

The data presented in Figures 6 and 7 looks at whether disease counts accurately reflect harvest seed quality. The data shows examples of bacteriophage treatment of potato seed leading to reduced bacterial loading in the progeny tubers over 2 generations: keeping potato seed clean of Pectobacterium (blackleg-causing bacteria) is very important, since very small amounts of disease (<0.5%) means that the crop can be downgraded, which represents a financial loss to the grower. In Figure 6, a potato crop was treated with bacteriophage either at planting (in-furrow) or with a weekly foliar spray or a combination of both treatments in 2016. qPCR on the harvested progeny tubers showed that the "in-furrow" & "in-furrow & foliar" treatment had significantly lower Pectobacterium than the control. These progeny tubers were replanted in 2017 (Figure 7), with no further treatments and less blackleg was seen in all of the treatments using seed from the 2016 phage treatments and in addition, the progeny tubers of the 2017 trial also had lower bacterial loading - i.e. a carryover effect from the 2016 treatments.

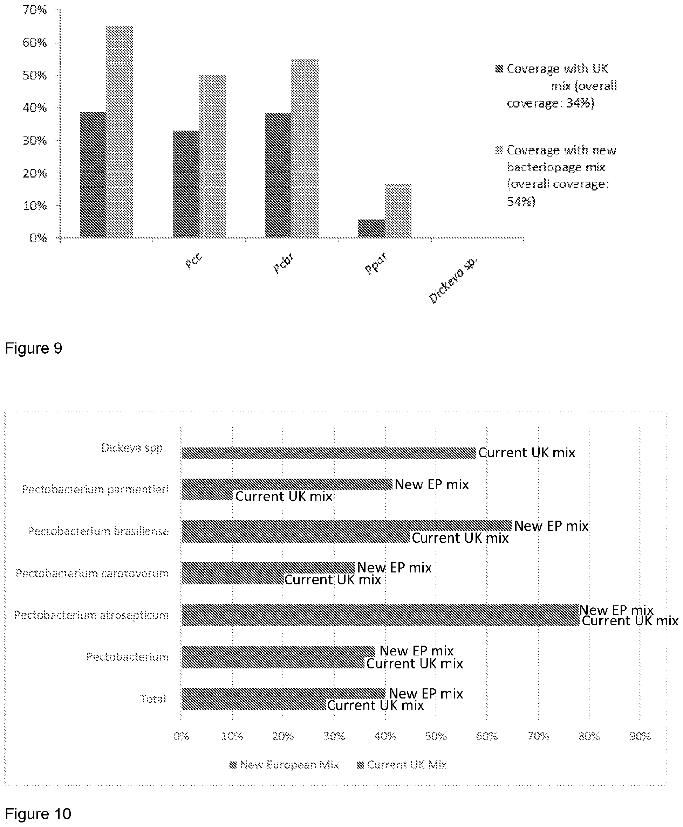

**Table 10 (above): current UK bacteriophage mix vs new European mix: % coverage of Pectobacterium spp. UK mix = 8 UK phage mix ( composition comprising, consisting essentially of or consisting of:APSPb1 (deposited as NCIMB 42290); APSPb5 (deposited as NCIMB 42292); APSPb2 (deposited as NCIMB 42294); APSPb6 (deposited as NCIMB 42296); APSPb3 (deposited as NCIMB 42298); APSPb4 (deposited as NCIMB 42300); APSPb7 (deposited as NCIMB 42302); and APSDs1 (deposited as NCIMB 42304)). New European mix = APS PH9 (Accession no: NCIMB 43321; APS PH10 (Accession no: NCIMB 43323; APS PH11 (Accession no: NCIMB 43325); APS PH12 (Accession no: NCIMB 43327; APS Ph3 (Accession No: NCIMB 42294); and APS Ph7 (Accession No: NCIMB 42302). Also see Figure 10.**

| | **Current UK (8 phage) Mix** | **New European Mix** |
|---|---|---|
| Total | 28% | 40% |
| Pectobacterium | 36% | 38% |
| Pectobacterium atrosepticum | 78% | 78% |
| Pectobacterium carotovorum | 20% | 34% |
| Pectobacterium brasiliense | 45% | 65% |
| Pectobacterium parmentieri | 10% | 41% |
| Dickeya spp. | 58% | 0% |

## Claims

1. A method of decontaminating food of one or more microorganisms, said method comprising contacting the food with one or more phages,
wherein the phages are selected from the group consisting of:
(i) a phage deposited as NCIMB 42290;
(ii) a phage deposited as NCIMB 42292;
(iii) a phage deposited as NCIMB 42294;
(iv) a phage deposited as NCIMB 42296;
(v) a phage deposited as NCIMB 42298;
(vi) a phage deposited as NCIMB 42300;
(vii) a phage deposited as NCIMB 42302;
(viii) a phage deposited as NCIMB 42304;
(ix) a phage deposited as NCIMB 43321;
(x) a phage deposited as NCIMB 43323;
(xi) a phage deposited as NCIMB 43325;
(xii) a phage deposited as NCIMB 43327;
(xiii) a phage deposited as NCIMB 42520;
(xiv) a phage deposited as NCIMB 42522;
(xv) a phage deposited as NCIMB 42524;
(xvi) a phage deposited as NCIMB 42526; and
(xvii) a phage deposited as NCIMB 42528.

2. A phage for use in a method of decontaminating food of one or more microorganisms, wherein the phage is selected from the group consisting of:
(i) a phage deposited as NCIMB 42290;
(ii) a phage deposited as NCIMB 42292;
(iii) a phage deposited as NCIMB 42294;
(iv) a phage deposited as NCIMB 42296;
(v) a phage deposited as NCIMB 42298;
(vi) a phage deposited as NCIMB 42300;
(vii) a phage deposited as NCIMB 42302;
(viii) a phage deposited as NCIMB 42304;
(ix) a phage deposited as NCIMB 43321;
(x) a phage deposited as NCIMB 43323;
(xi) a phage deposited as NCIMB 43325;
(xii) a phage deposited as NCIMB 43327;
(xiii) a phage deposited as NCIMB 42520;
(xiv) a phage deposited as NCIMB 42522;
(xv) a phage deposited as NCIMB 42524;
(xvi) a phage deposited as NCIMB 42526; and
(xvii) a phage deposited as NCIMB 42528.

3. A composition for the decontamination of food of one or more microorganisms, said composition comprising one or more phages, wherein the phages are selected from the group consisting of:
(i) a phage deposited as NCIMB 42290;
(ii) a phage deposited as NCIMB 42292;
(iii) a phage deposited as NCIMB 42294;
(iv) a phage deposited as NCIMB 42296;
(v) a phage deposited as NCIMB 42298;
(vi) a phage deposited as NCIMB 42300;
(vii) a phage deposited as NCIMB 42302;
(viii) a phage deposited as NCIMB 42304;
(ix) a phage deposited as NCIMB 43321;
(x) a phage deposited as NCIMB 43323;
(xi) a phage deposited as NCIMB 43325;
(xii) a phage deposited as NCIMB 43327;
(xiii) a phage deposited as NCIMB 42520;
(xiv) a phage deposited as NCIMB 42522;
(xv) a phage deposited as NCIMB 42524;
(xvi) a phage deposited as NCIMB 42526; and
(xvii) a phage deposited as NCIMB 42528.

4. The method of claim 1, wherein the food is selected from the group consisting of:
(i) any food, food stuff and/or food product for human or animal consumption;
(ii) manufactured, harvested, farmed or processed food, food stuff, produce or product;
(iii) crops;
(iv) cereals;
(v) dairy produce;
(vi) meat;
(vii) vegetables and root vegetables;
(viii) edible leaves, herbs or salad;
(ix) fruit;
(x) seeds;
(xi) tubers;
(xii) bagged salad; and
(xiii) potatoes.

5. The method of claim 1 or 4, wherein the microorganism(s) is/are part of the microbial flora of the food.

6. The method of claim 1 or 4 to 5, wherein the microorganism(s) is/are a microbial contaminant or pathogens of the food.

7. The method of claim 1 or 4 to 6, wherein the microorganism(s) is/are associated with spoilage, premature ripening and/or rotting of the food.

8. The method of claim 1 or 4 to 7, phage of claim 2, or composition of claim 3, wherein the method, phage or composition comprise one or more phage types specific for one or more microorganisms.

9. The method of any one of claims 1 or 4 to 8, phage of claim 2 or 8, or composition of claims 3 or 8, wherein the phages comprise:
(i) a phage deposited as NCIMB 42290;
(ii) a phage deposited as NCIMB 42292;
(iii) a phage deposited as NCIMB 42294;
(iv) a phage deposited as NCIMB 42296;
(v) a phage deposited as NCIMB 42298;
(vi) a phage deposited as NCIMB 42300;
(vii) a phage deposited as NCIMB 42302; and
(viii) a phage deposited as NCIMB 42304.

10. The method of any one of claims 1 or 4 to 8, phage of claim 2 or 8, or composition of claim 3 or 8, wherein the phages are selected from the group consisting of:
(i) a phage deposited as NCIMB 43321;
(ii) a phage deposited as NCIMB 43323;
(iii) a phage deposited as NCIMB 43325; and
(iv) a phage deposited as NCIMB 43327.

11. The method of any one of claims 1 or 4 to 8, phage of claim 2 or 8 or composition of claim 3 or 8, wherein the phages comprise:
(i) a phage deposited as NCIMB 43321;
(ii) a phage deposited as NCIMB 43323;
(iii) a phage deposited as NCIMB 43325; and
(iv) a phage deposited as NCIMB 43327.

12. The method, phage or composition of claim 11, wherein the phages further comprises the phage deposited as NCIMB 42294 and/or the phage deposited as Accession No: NCIMB 42302.

13. Food and/or stored or packed food comprising one or more phages as defined in claims 1-3, wherein the food has been treated with:
(i) the method of any one of claims 1 or 4 to 12;
(ii) the phage of any one of claims 2 or 8 to 12; or
(iii) the composition of any one of claims 3 or 8 to 12.

## Patentansprüche

1. Verfahren zur Dekontamination von Lebensmitteln von einem Mikroorganismus oder mehreren Mikroorganismen, wobei das Verfahren das In-Berührung-Bringen des Nahrungs-/Lebensmittels mit einem oder mehreren Phagen umfasst,
wobei die Phagen aus der Gruppe ausgewählt sind bestehend aus:
(i) einem Phagen, der als NCIMB 42290 hinterlegt worden ist;
(ii) einem Phagen, der als NCIMB 42292 hinterlegt worden ist;
(iii) einem Phagen, der als NCIMB 42294 hinterlegt worden ist;
(iv) einem Phagen, der als NCIMB 42296 hinterlegt worden ist;
(v) einem Phagen, der als NCIMB 42298 hinterlegt worden ist;
(vi) einem Phagen, der als NCIMB 42300 hinterlegt worden ist;
(vii) einem Phagen, der als NCIMB 42302 hinterlegt worden ist;
(viii) einem Phagen, der als NCIMB 42304 hinterlegt worden ist;
(ix) einem Phagen, der als NCIMB 43321 hinterlegt worden ist;
(x) einem Phagen, der als NCIMB 43323 hinterlegt worden ist;
(xi) einem Phagen, der als NCIMB 43325 hinterlegt worden ist;
(xii) einem Phagen, der als NCIMB 43327 hinterlegt worden ist;
(xiii) einem Phagen, der als NCIMB 42520 hinterlegt worden ist;
(xiv) einem Phagen, der als NCIMB 42522 hinterlegt worden ist;
(xv) einem Phagen, der als NCIMB 42524 hinterlegt worden ist;
(xvi) einem Phagen, der als NCIMB 42526 hinterlegt worden ist; und
(xvii) einem Phagen, der als NCIMB 42528 hinterlegt worden ist.

2. Phage zur Verwendung in einem Verfahren zur Dekontamination von Lebensmitteln von einem Mikroorganismus oder mehreren Mikroorganismen, wobei der Phage aus der Gruppe ausgewählt ist bestehend aus:
(i) einem Phagen, der als NCIMB 42290 hinterlegt worden ist;
(ii) einem Phagen, der als NCIMB 42292 hinterlegt worden ist;
(iii) einem Phagen, der als NCIMB 42294 hinterlegt worden ist;
(iv) einem Phagen, der als NCIMB 42296 hinterlegt worden ist;
(v) einem Phagen, der als NCIMB 42298 hinterlegt worden ist;
(vi) einem Phagen, der als NCIMB 42300 hinterlegt worden ist;
(vii) einem Phagen, der als NCIMB 42302 hinterlegt worden ist;
(viii) einem Phagen, der als NCIMB 42304 hinterlegt worden ist;
(ix) einem Phagen, der als NCIMB 43321 hinterlegt worden ist;
(x) einem Phagen, der als NCIMB 43323 hinterlegt worden ist;
(xi) einem Phagen, der als NCIMB 43325 hinterlegt worden ist;
(xii) einem Phagen, der als NCIMB 43327 hinterlegt worden ist;
(xiii) einem Phagen, der als NCIMB 42520 hinterlegt worden ist;
(xiv) einem Phagen, der als NCIMB 42522 hinterlegt worden ist;
(xv) einem Phagen, der als NCIMB 42524 hinterlegt worden ist;
(xvi) einem Phagen, der als NCIMB 42526 hinterlegt worden ist; und
(xvii) einem Phagen, der als NCIMB 42528 hinterlegt worden ist.

3. Zusammensetzung zur Dekontamination von Lebensmitteln von einem Mikroorganismus oder mehreren Mikroorganismen, wobei die Zusammensetzung einen oder mehrere Phagen umfasst, wobei die Phagen aus der Gruppe ausgewählt sind bestehend aus:
(i) einem Phagen, der als NCIMB 42290 hinterlegt worden ist;
(ii) einem Phagen, der als NCIMB 42292 hinterlegt worden ist;
(iii) einem Phagen, der als NCIMB 42294 hinterlegt worden ist;
(iv) einem Phagen, der als NCIMB 42296 hinterlegt worden ist;
(v) einem Phagen, der als NCIMB 42298 hinterlegt worden ist;
(vi) einem Phagen, der als NCIMB 42300 hinterlegt worden ist;
(vii) einem Phagen, der als NCIMB 42302 hinterlegt worden ist;
(viii) einem Phagen, der als NCIMB 42304 hinterlegt worden ist;
(ix) einem Phagen, der als NCIMB 43321 hinterlegt worden ist;
(x) einem Phagen, der als NCIMB 43323 hinterlegt worden ist;
(xi) einem Phagen, der als NCIMB 43325 hinterlegt worden ist;
(xii) einem Phagen, der als NCIMB 43327 hinterlegt worden ist;
(xiii) einem Phagen, der als NCIMB 42520 hinterlegt worden ist;
(xiv) einem Phagen, der als NCIMB 42522 hinterlegt worden ist;
(xv) einem Phagen, der als NCIMB 42524 hinterlegt worden ist;
(xvi) einem Phagen, der als NCIMB 42526 hinterlegt worden ist; und
(xvii) einem Phagen, der als NCIMB 42528 hinterlegt worden ist.

4. Verfahren nach Anspruch 1, wobei das Lebensmittel aus der Gruppe ausgewählt ist bestehend aus:
(i) irgendeinem Nahrungs-/Lebensmittel, Nährmittel und/oder Nahrungs- /Lebensmittelprodukt für den menschlichen oder tierischen Verzehr;
(ii) gefertigtem, geerntetem, landwirtschaftlich erzeugtem oder verarbeitetem Nahrungs-/Lebensmittel, Nährmittel, Erzeugnis oder Produkt;
(iii) Nutzpflanzen;
(iv) Zerealien
(v) Molkereiprodukt;
(vi) Fleisch;
(vii) Gemüsen und Wurzelgemüsen;
(viii) essbaren Blättern, Kräutern oder Salat;
(ix) Obst;
(x) Samen;
(xi) Knollenfrüchten;
(xii) Beutelsalat; und
(xiii) Kartoffeln.

5. Verfahren nach Anspruch 1 oder 4, wobei der Mikroorganismus/die Mikroorganismen Teil der Mikrobenflora des Lebensmittels ist/sind.

6. Verfahren nach Anspruch 1 oder 4 bis 5, wobei der Mikroorganismus/die Mikroorganismen ein mikrobieller Kontaminant oder Pathogene des Lebensmittels ist/sind.

7. Verfahren nach Anspruch 1 oder 4 bis 6, wobei der Mikroorganismus/die Mikroorganismen mit Verderb, vorzeitiger Reifung und/oder Verfaulen des Nahrungs-/Lebensmittels verbunden ist/sind

8. Verfahren nach Anspruch 1 oder 4 bis 7, Phage nach Anspruch 2 oder Zusammensetzung nach Anspruch 3, wobei das Verfahren, der Phage oder die Zusammensetzung einen oder mehrere Phagentypen, der/die für einen Mikroorganismus oder mehrere Mikroorganismen spezifisch ist/sind, umfasst.

9. Verfahren nach einem der Ansprüche 1 oder 4 bis 8, Phage nach Anspruch 2 oder 8 oder Zusammensetzung nach Anspruch 3 oder 8, wobei die Phagen Folgendes umfassen:
(i) einen Phagen, der als NCIMB 42290 hinterlegt worden ist;
(ii) einen Phagen, der als NCIMB 42292 hinterlegt worden ist;
(iii) einen Phagen, der als NCIMB 42294 hinterlegt worden ist;
(iv) einen Phagen, der als NCIMB 42296 hinterlegt worden ist;
(v) einen Phagen, der als NCIMB 42298 hinterlegt worden ist;
(vi) einen Phagen, der als NCIMB 42300 hinterlegt worden ist;
(vii) einen Phagen, der als NCIMB 42302 hinterlegt worden ist; und
(viii) einen Phagen, der als NCIMB 42304 hinterlegt worden ist;

10. Verfahren nach einem der Ansprüche 1 oder 4 bis 8, Phage nach Anspruch 2 oder 8 oder Zusammensetzung nach Anspruch 3 oder 8, wobei die Phagen aus der Gruppe ausgewählt sind bestehend aus:
(i) einem Phagen, der als NCIMB 43321 hinterlegt worden ist;
(ii) einem Phagen, der als NCIMB 43323 hinterlegt worden ist;
(iii) einem Phagen, der als NCIMB 43325 hinterlegt worden ist; und
(iv) einem Phagen, der als NCIMB 43327 hinterlegt worden ist.

11. Verfahren nach einem der Ansprüche 1 oder 4 bis 8, Phage nach Anspruch 2 oder 8 oder Zusammensetzung nach Anspruch 3 oder 8, wobei die Phagen Folgendes umfassen:
(i) einen Phagen, der als NCIMB 43321 hinterlegt worden ist;
(ii) einen Phagen, der als NCIMB 43323 hinterlegt worden ist;
(iii) einen Phagen, der als NCIMB 43325 hinterlegt worden ist; und
(iv) einen Phagen, der als NCIMB 43327 hinterlegt worden ist.

12. Verfahren, Phage oder Zusammensetzung nach Anspruch 11, wobei die Phagen ferner den Phagen, der als NCIMB 42294 hinterlegt worden ist, und/oder den Phagen, der als Zugangsnummer NCIMB 42302 hinterlegt worden ist, umfassen.

13. Lebensmittel und/oder gelagertes oder verpacktes /Lebensmittel umfassend einen oder mehrere Phagen, wie in den Ansprüchen 1-3 definiert, wobei das Lebensmittel behandelt worden ist mit:
(i) dem Verfahren nach einem der Ansprüche 1 oder 4 bis 12;
(ii) dem Phagen nach einem der Ansprüche 2 oder 8 bis 12; oder
(iii) der Zusammensetzung nach einem der Ansprüche 3 oder 8 bis 12.

## Revendications

1. Procédé de décontamination d'un aliment d'un ou de plusieurs micro-organismes, ledit procédé comprenant la mise en contact de l'aliment avec un ou plusieurs phages,
dans lequel les phages sont sélectionnés dans le groupe constitué de :
(i) un phage déposé en tant que NCIMB 42290,
(ii) un phage déposé en tant que NCIMB 42292,
(iii) un phage déposé en tant que NCIMB 42294,
(iv) un phage déposé en tant que NCIMB 42296,
(v) un phage déposé en tant que NCIMB 42298,
(vi) un phage déposé en tant que NCIMB 42300,
(vii) un phage déposé en tant que NCIMB 42302,
(viii) un phage déposé en tant que NCIMB 42304,
(ix) un phage déposé en tant que NCIMB 43321,
(x) un phage déposé en tant que NCIMB 43323,
(xi) un phage déposé en tant que NCIMB 43325,
(xii) un phage déposé en tant que NCIMB 43327,
(xiii) un phage déposé en tant que NCIMB 42520,
(xiv) un phage déposé en tant que NCIMB 42522,
(xv) un phage déposé en tant que NCIMB 42524,
(xvi) un phage déposé en tant que NCIMB 42526, et
(xvii) un phage déposé en tant que NCIMB 42528.

2. Phage pour une utilisation dans un procédé de décontamination d'un un aliment d'un ou de plusieurs micro-organismes, où le phage est sélectionné dans le groupe constitué de :
(i) un phage déposé en tant que NCIMB 42290,
(ii) un phage déposé en tant que NCIMB 42292,
(iii) un phage déposé en tant que NCIMB 42294,
(iv) un phage déposé en tant que NCIMB 42296,
(v) un phage déposé en tant que NCIMB 42298,
(vi) un phage déposé en tant que NCIMB 42300,
(vii) un phage déposé en tant que NCIMB 42302,
(viii) un phage déposé en tant que NCIMB 42304,
(ix) un phage déposé en tant que NCIMB 43321,
(x) un phage déposé en tant que NCIMB 43323,
(xi) un phage déposé en tant que NCIMB 43325,
(xii) un phage déposé en tant que NCIMB 43327,
(xiii) un phage déposé en tant que NCIMB 42520,
(xiv) un phage déposé en tant que NCIMB 42522,
(xv) un phage déposé en tant que NCIMB 42524,
(xvi) un phage déposé en tant que NCIMB 42526, et
(xvii) un phage déposé en tant que NCIMB 42528.

3. Composition pour la décontamination d'un aliment d'un ou de plusieurs micro-organismes, ladite composition comprenant un ou plusieurs phages, dans laquelle les phages sont sélectionnés dans le groupe constitué de :
(i) un phage déposé en tant que NCIMB 42290,
(ii) un phage déposé en tant que NCIMB 42292,
(iii) un phage déposé en tant que NCIMB 42294,
(iv) un phage déposé en tant que NCIMB 42296,
(v) un phage déposé en tant que NCIMB 42298,
(vi) un phage déposé en tant que NCIMB 42300,
(vii) un phage déposé en tant que NCIMB 42302,
(viii) un phage déposé en tant que NCIMB 42304,
(ix) un phage déposé en tant que NCIMB 43321,
(x) un phage déposé en tant que NCIMB 43323,
(xi) un phage déposé en tant que NCIMB 43325,
(xii) un phage déposé en tant que NCIMB 43327,
(xiii) un phage déposé en tant que NCIMB 42520,
(xiv) un phage déposé en tant que NCIMB 42522,
(xv) un phage déposé en tant que NCIMB 42524,
(xvi) un phage déposé en tant que NCIMB 42526, et
(xvii) un phage déposé en tant que NCIMB 42528.

4. Procédé selon la revendication 1, dans lequel l'aliment est sélectionné dans le groupe constitué de :
(i) un quelconque aliment, denrée alimentaire et/ou produit alimentaire destiné à une consommation humaine ou animale,
(ii) un aliment, denrée alimentaire ou produit alimentaire transformé, récolté, d'élevage ou traité,
(iii) des végétaux,
(iv) des céréales,
(v) des produits laitiers,
(vi) de la viande,
(vii) des légumes et des légumes à racines,
(viii) des feuilles et herbes comestibles ou des salades,
(ix) des fruits,
(x) des graines,
(xi) des tubercules,
(xii) de la salade en sachet, et
(xiii) des pommes de terre.

5. Procédé selon la revendication 1 ou 4, dans lequel le(s) micro-organisme(s) fait/font partie de la flore microbienne de l'aliment.

6. Procédé selon la revendication 1 ou 4 à 5, dans lequel le(s) micro-organisme(s) est/sont un contaminant microbien ou des agents pathogènes de l'aliment.

7. Procédé selon la revendication 1 ou 4 à 6, dans lequel le(s) micro-organisme(s) est/sont associé(s) à une altération, une maturation prématurée et/ou une putréfaction de l'aliment.

8. Procédé selon la revendication 1 ou de 4 à 7, phage selon la revendication 2 ou composition selon la revendication 3, où le procédé, le phage ou la composition comprennent un ou plusieurs types de phage spécifiques à un ou plusieurs micro-organismes.

9. Procédé selon l'une quelconque des revendications 1 ou 4 à 8, phage selon la revendication 2 ou 8 ou composition selon les revendications 3 ou 8, dans lequel(laquelle) les phages comprennent :
(i) un phage déposé en tant que NCIMB 42290,
(ii) un phage déposé en tant que NCIMB 42292,
(iii) un phage déposé en tant que NCIMB 42294,
(iv) un phage déposé en tant que NCIMB 42296,
(v) un phage déposé en tant que NCIMB 42298,
(vi) un phage déposé en tant que NCIMB 42300,
(vii) un phage déposé en tant que NCIMB 42302, et
(viii) un phage déposé en tant que NCIMB 42304.

10. Procédé selon l'une quelconque des revendications 1 ou 4 à 8, phage selon la revendication 2 ou 8 ou composition selon la revendication 3 ou 8, dans lequel(laquelle) les phages sont sélectionnés dans le groupe constitué de :
(i) un phage déposé en tant que NCIMB 43321,
(ii) un phage déposé en tant que NCIMB 43323,
(iii) un phage déposé en tant que NCIMB 43325, et
(iv) un phage déposé en tant que NCIMB 43327.

11. Procédé selon l'une quelconque des revendications 1 ou 4 à 8, phage selon la revendication 2 ou 8 ou composition selon la revendication 3 ou 8, dans lequel(laquelle) les phages comprennent :
(i) un phage déposé en tant que NCIMB 43321,
(ii) un phage déposé en tant que NCIMB 43323,
(iii) un phage déposé en tant que NCIMB 43325, et
(iv) un phage déposé en tant que NCIMB 43327.

12. Procédé, phage ou composition selon la revendication 11, dans lequel(laquelle) les phages comprennent en outre le phage déposé en tant que NCIMB 42294 et/ou le phage déposé sous le numéro d'ordre NCIMB 42302.

13. Aliment et/ou aliment stocké ou emballé comprenant un ou plusieurs phages tels que définis selon les revendications 1 à 3, où l'aliment a été traité avec :
(i) le procédé selon l'une quelconque des revendications 1 ou 4 à 12,
(ii) le phage selon l'une quelconque des revendications 2 ou 8 à 12, ou
(iii) la composition selon l'une quelconque des revendications 3 ou 8 à 12.
